# EUROPÄISCHE PATENTSCHRIFT

(11) **EP 4 243 740 B1**
(45) Veröffentlichungstag und Bekanntmachung des Hinweises auf die Patenterteilung: **23.07.2025**
(21) Anmeldenummer: 21810340.6
(22) Anmeldetag: 10.11.2021
(51) Int. Cl.: A61F 2/64, A61F 2/74

(54) **DÄMPFEREINHEIT UND ORTHOPÄDIETECHNISCHE VORRICHTUNG**
DAMPER UNIT AND ORTHOPAEDIC DEVICE
UNITÉ D'AMORTISSEMENT ET DISPOSITIF ORTHOPÉDIQUE

(30) Priorität: 12.11.2020 DE 102020129853
(43) Veröffentlichungstag der Anmeldung: 20.09.2023
(73) Patentinhaber: Otto Bock Healthcare Products GmbH, 1110 Wien (AT)
(72) Erfinder: EDER, Florian, 1040 Wien (AT)
(74) Vertreter: Gramm, Lins & Partner Patent- und Rechtsanwälte PartGmbB
(86) Internationale Anmeldenummer: PCT/EP2021/081215
(87) Internationale Veröffentlichungsnummer: WO 2022/101254

(56) Entgegenhaltungen:
- EP-B1- 3 374 587
- DE-A1- 102016 118 999
- US-A1- 2004 061 303

## Beschreibung

Die Erfindung betrifft eine Dämpfereinheit mit einem Hydraulikzylinder mit einem verschieblich darin gelagerten Hydraulikkolben, der mit einer Kolbenstange gekoppelt ist und den Hydraulikzylinder in zwei Hydraulikkammern unterteilt, die über zumindest einen Hydraulikkanal miteinander strömungstechnisch verbunden sind, und mit einem Pneumatikzylinder mit einem verschieblich darin gelagerten Pneumatikkolben, der mit der Kolbenstange gekoppelt ist und den Pneumatikzylinder in zwei Pneumatikkammern unterteilt, die über zumindest einen Pneumatikkanal miteinander strömungstechnisch verbunden sind. Die Erfindung betrifft ebenfalls eine orthopädietechnische Vorrichtung mit einem Oberteil und einem Unterteil, die über ein Gelenk schwenkbar aneinander gelagert sind, und einer Dämpfereinheit, wie sie oben beschrieben ist. Die orthopädietechnische Vorrichtung ist insbesondere als eine Orthese, ein Exoskelett oder eine Prothese ausgebildet, insbesondere als ein künstliches Kniegelenk, ist jedoch nicht auf solche Anwendungsfälle beschränkt

Um beispielsweise ein Bewegungsverhalten zweier Komponenten zu steuern, werden Relativbewegungen zwischen zwei Komponenten über Dämpfereinrichtungen beeinflusst. Die jeweils zwischen den beiden Komponenten angeordnete Dämpfereinrichtung stellt einen Widerstand gegen die Relativbewegung bereit, wobei dieser Widerstand eingestellt werden kann. Eine häufige Ausgestaltung der Dämpfereinrichtung sieht einen linearen Hydraulikdämpfer vor, der in einem Gehäuse angeordnet ist. Innerhalb des Gehäuses ist ein Zylinder ausgebildet oder angeordnet, in dem ein Kolben, der an einer Kolbenstange angeordnet ist, verlagert wird. Die Kolbenstange ist mit einer ersten Komponente gekoppelt, das Gehäuse bzw. der Zylinder mit der zweiten Komponente. Der Zylinder ist mit einem Hydraulikfluid befüllt, das bei einer Kolbenbewegung von der sich verkleinerten Hydraulikkammer in die sich vergrößernde Hydraulikkammer gefördert wird. Die Förderung kann entweder durch den Kolben hindurch oder durch einen oder mehrere Hydraulikkanäle innerhalb des Gehäuses erfolgen. In dem Hydraulikkanal oder in den Hydraulikkanälen sind Drosseleinrichtungen vorhanden, um den Strömungswiderstand zu erhöhen bzw. einzustellen. Die Drosseleinrichtungen können verstellbar ausgebildet sein, beispielsweise in Gestalt von Stellventilen oder Schaltventilen, sodass ein veränderlicher Strömungswiderstand bereitgestellt werden kann. Verstellbare Drosseleinrichtungen sind in der Regel nicht in einem Kolben angeordnet. Eine hydraulische Dämpfereinrichtung ist beispielsweise aus der EP 2 285 315 B1 bekannt.

Neben hydraulischen Dämpfereinrichtungen sind pneumatische Dämpfereinrichtungen bekannt, die einen gleichen konstruktiven Aufbau wie hydraulische Dämpfereinrichtungen aufweisen. Aus der DE 10 2016 118 999 A1 ist eine Aktuator-Dämpfereinheit zum Einsatz in orthetischen und prothetischen Vorrichtungen bekannt, bei dem in einem Gehäuse ein Zylinder ausgebildet ist, in dem ein erster Kolben verlagerbar gelagert und mit einer Kolbenstange gekoppelt ist. Zumindest ein weiterer Kolben ist mit dem ersten Kolben zur Ausbildung einer weiteren, volumenveränderbaren Fluidkammer gekoppelt, die Fluidkammer kann beispielsweise als pneumatische Feder ausgebildet sein.

Die US 6 517 582 B1 betrifft ein Prothesenbein mit einem Prothesenkniegelenk und einer hydraulischen Widerstandseinrichtung, die über eine sensorgesteuerte Steuervorrichtung hinsichtlich des hydraulischen Widerstandes einstellbar ist. Zusätzlich zu der hydraulischen Widerstandseinrichtung ist eine pneumatische Widerstandseinrichtung vorgesehen. Die Steuervorrichtung kann den hydraulischen und pneumatischen Widerstand gegen eine Kniebeugung einstellen.

Die US 2004/0061303 A1 betrifft ein Fahrrad mit einem Rahmen und einer Einstellvorrichtung zum Einstellen der Höhe eines Sattels oder einer Lenkstange. Die Einstellvorrichtung weist eine doppelt wirkende Kolben-Zylinder-Anordnung auf. Mit einer Kolbenstange sind ein Hydraulikkolben und ein Pneumatikkolben verbunden, die jeweils einen Zylinder in zwei Kammern aufteilen.

Die DE 10 2016 118 999 A1 betrifft eine Aktuator-Dämpfer-Einheit zum Einsatz in orthetischen oder prothetischen Vorrichtungen mit einem Zylinder, in dem ein erster Kolben verlagerbar gelagert und mit einer Kolbenstange gekoppelt ist, die mit einem ersten Ende an dem ersten Kolben angeordnet und mit einem zweiten Ende mit der orthetischen oder prothetischen Vorrichtung koppelbar ist. Der erste Kolben trennt zwei Fluidkammern in dem Zylinder voneinander. Ein weiterer Kolben ist mit dem ersten Kolben zur Ausbildung zumindest einer weiteren volumenveränderbaren Fluidkammer gekoppelt.

Die EP 3 374 587 B1 beschreibt eine Türkomponente mit einer steuerbaren Dämpfereinrichtung. Die Dämpfereinrichtung umfasst eine Zylindereinheit, in der eine Kolbeneinheit zwei Kammern voneinander trennt. Die betraglich unterschiedlichen Volumenänderungen in den Kammern werden durch ein Ausgleichsvolumen ausgeglichen.

Aufgabe der vorliegenden Erfindung ist es, eine Dämpfereinheit und eine orthopädietechnische Vorrichtung bereitzustellen, mit denen es vereinfacht möglich ist, einen Widerstand gegen eine erste Bewegung und bei Bewegungsrichtungsumkehr eine Unterstützung bereitzustellen.

Erfindungsgemäß wird diese Aufgabe durch eine Dämpfereinheit und eine orthopädietechnische Vorrichtung mit den Merkmalen der unabhängigen Ansprüche gelöst. Vorteilhafte Ausgestaltungen und Weiterbildungen der Erfindung sind in den Unteransprüchen, der Beschreibung sowie den Figuren offenbart.

Die Dämpfereinheit mit einem Hydraulikzylinder mit einem verschieblich darin gelagerten Hydraulikkolben, der mit einer Kolbenstange gekoppelt ist und den Hydraulikzylinder in zwei Hydraulikkammern unterteilt, die über zumindest einen Hydraulikkanal miteinander strömungstechnisch verbunden sind, und mit einem Pneumatikzylinder mit einem verschieblich darin gelagerten Pneumatikkolben, der mit der Kolbenstange gekoppelt ist und den Pneumatikzylinder in zwei Pneumatikkammern unterteilt, die über zumindest einen Pneumatikkanal miteinander strömungstechnisch verbunden sind, sieht vor, dass der Betrag der Volumenänderung der Hydraulikkammern bei einer Verlagerung des Hydraulikkolbens verschieden ist und die Hydraulikkammern mit einem Ausgleichsvolumen strömungstechnisch gekoppelt sind. Durch die Kopplung des Hydraulikzylinders in der Dämpfereinheit mit einem Pneumatikzylinder ist es möglich, eine hydraulisch gedämpfte Bewegung um eine Gasfeder oder eine Pneumatikfeder zu ergänzen. Während der bremsenden Bewegung durch den Hydraulikzylinder ist es gleichzeitig möglich, durch Kompression in einer Pneumatikkammer das darin befindliche Gasvolumen oder das darin befindliche Volumen einer kompressiblen Flüssigkeit zu spannen und bei einer Bewegungsumkehr in entgegengesetzte Richtung die Kompressionsenergie wieder freizugeben, um so eine entgegengesetzt gerichtete Bewegung von Hydraulikzylinder und Pneumatikzylinder und damit auch der Kolbenstange zu bewirken oder zu unterstützen. Die hydraulische Dämpfung wird durch die bremsende Phase beim Aufladen der Pneumatikkammer unterstützt und die gespeicherte Bewegungsenergie unterstützt beim Entspannen des komprimierten Gases oder der komprimierten kompressiblen Flüssigkeit die Bewegung in umgekehrter Richtung. Insbesondere bei der Anwendung der Dämpfereinheit in einem künstlichen Kniegelenk, beispielsweise einem Prothesenkniegelenk oder Orthesenkniegelenk, wird das federnde Einbeugen zu Beginn der Standphase, die sogenannte Standphasenbeugung, ermöglicht und die Standphasenstreckung unterstützt. Wird nachfolgend von komprimierbaren oder komprimierten Gasen gesprochen, gelten diese Ausführungen ebenso für kompressible Flüssigkeiten.

Die Erfindung sieht vor, dass der Betrag der Volumenänderung der Pneumatikkammern bei einer Verlagerung des Pneumatikkolbens gleich ist. Dies wird insbesondere dadurch erreicht, dass beide Seiten des Pneumatikkolbens die gleichen wirksamen Flächen aufweisen. Durch die gleichgroßen wirksamen Kolbenflächen und die gleichen Beträge der sich ändernden Volumina in den beiden Pneumatikkammern ist es möglich, die Pneumatik über den Pneumatikkanal unwirksam werden zu lassen, sodass keine Kräfte der Pneumatik bei einem offenen Pneumatikkanal auftreten. Dies bedeutet, dass die Pneumatik mit sehr viel höheren Drücken befüllbar ist, ohne eine ungewollte Vorbringerwirkung zu entwickeln, wodurch eine hohe Aufladung der komprimierten Pneumatikkammer erreicht werden kann und dadurch hohe Kräfte aufgenommen und aufgebracht werden können.

Der Pneumatikkolben ist in einer Ausgestaltung an der Kolbenstange angeordnet, wobei die Kolbenstange den Pneumatikzylinder durchragt. Dabei muss die Kolbenstange kein einstückiges Bauteil sein, es besteht ebenfalls die Möglichkeit, dass zwei einander gegenüberliegende Kolbenstangenabschnitte auf gegenüberliegenden Seiten des Pneumatikkolbens angeordnet bzw. befestigt sind. Durch die durchgehende Kolbenstange mit gleichen Querschnitten werden die gleichen wirksamen Kolbenflächen in dem Pneumatikzylinder bereitgestellt.

**In** einer Variante ist der Pneumatikkolben an dem Hydraulikzylinder befestigt, wobei der Hydraulikzylinder beweglich in dem Pneumatikzylinder angeordnet ist. Bei dieser Ausgestaltung befindet sich der Hydraulikkolben innerhalb des Hydraulikzylinders, der Pneumatikkolben hingegen ist an der Außenseite des Hydraulikzylinders angeordnet und wird gemeinsam mit dem Hydraulikzylinder innerhalb des Pneumatikyzlinders verschoben. Dies führt zu einer Verringerung der Bauhöhe aufgrund des ineinander geschachtelten Aufbaus. Dabei wird in Kauf genommen, dass aufgrund der unterschiedlichen Kolbenstangendurchmesser unterschiedliche pneumatische Kräfte bei einem geöffneten Pneumatikkanal entstehen. Für bestimmte Anwendungen kann dies jedoch durchaus erwünscht sein.

**In** einer Weiterbildung ist in dem Hydraulikkanal zumindest ein schaltbares oder verstellbares Hydraulikventil angeordnet, mit dem oder denen es möglich ist, den hydraulischen Widerstand einzustellen. Insbesondere erfolgt die Verstellung oder das Schalten des Hydraulikventils auf der Grundlage von Sensordaten, die an eine Steuervorrichtung übermittelt werden, die wiederum einen Aktuator zum Öffnen, Schließen oder Verstellen des Hydraulikventils aktiviert und/oder deaktiviert. Insbesondere sind zwei Stellventile in dem Hydraulikkanal oder in den Hydraulikkanälen angeordnet, eines zur Einstellung eines Flexionswiderstandes und das andere zur Einstellung des Extensionswiderstandes. Zwei parallel angeordnete Rückschlagventile und ein Verbindungskanal zu dem Ausgleichsvolumen können darüber hinaus vorgesehen sein, um einen an die jeweilige Situation angepassten, variablen hydraulischen Widerstand bereitstellen zu können.

In einer Ausgestaltung ist das Ausgleichsvolumen geschlossen ausgebildet und weist eine Luft- oder Gasblase auf, die beim Befüllen des Ausgleichsvolumens komprimiert wird. Dadurch wird das Hydraulikfluid durch das komprimierte Gas vorgespannt und kann durch eine entsprechende Ventilschaltung zum Antreiben oder Unterstützen der jeweils gewünschten Bewegung der Kolbenstange eingesetzt werden. Neben einer pneumatischen Federvorspannung des Hydraulikfluids kann diese auch mechanisch erfolgen, beispielsweise über einen federbelasteten Kolben innerhalb des Ausgleichsvolumens.

In dem Pneumatikkanal ist in einer Ausgestaltung der Erfindung zumindest ein schaltbares oder verstellbares Pneumatikventil angeordnet, über das die Pneumatik wirkungslos geschaltet werden kann. Bei einem geöffneten Pneumatikventil und bei gleichen wirksamen Flächen des Pneumatikkolbens ist es möglich, das in dem Pneumatiksystem befindliche Gas kraftlos zwischen den beiden Kammern zu verschieben. Die Pneumatik wird somit keinen wesentlichen Anteil an einem Widerstand gegen eine Verlagerung der Kolbenstange bieten. Unabhängig von dem Fülldruck im Pneumatiksystem ist es somit möglich, diese zuzuschalten oder abzuschalten. Wird das Pneumatikventil bei unterschiedlichen Kolbenstellungen geschlossen, lässt sich darüber der Nullpunkt der dann entstehenden Pneumatikfeder verschieben. Somit wird der Einsatz der Feder in unterschiedlichen Stellungen der Kolbenstange ermöglicht. Bei einem Einsatz der Dämpfereinrichtung bei einer unteren Extremität kann somit der Einsatz einer Pneumatikfeder in unterschiedlichen Gangphasen oder Bewegungsphasen ermöglicht werden. Neben dem Einsatz bei der Standphasenbeugung kann eine Pneumatikfeder beispielsweise auch zur Unterstützung der Extensionsbewegung am Ende der Schwungphasenbeugung eingesetzt werden. Somit ist eine Verschiebung des Nullpunktes der Feder bei einem nahezu gleichbleibenden Federverhalten aufgrund der gleichgroßen wirksamen Kolbenflächen einfach möglich. Die Verschiebung eines Nullpunktes der pneumatischen Feder kann somit durch das Öffnen und Schließen eines einzigen Pneumatikventils einfach eingestellt werden.

In einer Weiterbildung ist vorgesehen, dass in dem Pneumatikkanal zwei Pneumatikventile angeordnet sind, zu denen zwei einander entgegengesetzt geschaltete Rückschlagventile in einem Parallelkanal angeordnet sind, wobei der Parallelkanal mit dem Pneumatikkanal über einen Verbindungskanal zwischen den Pneumatikventilen und den Rückschlagventilen verbunden ist. Durch eine solche Verschaltung der Pneumatikkammern mit zwei Stellventilen bzw. Schaltventilen und zwei Rückschlagventilen ist es möglich, den Pneumatikteil der Dämpfereinrichtung im Vorfeld einer zu erwartenden Schaltung vorzubereiten und die jeweiligen Ventile zu öffnen bzw. zu schließen. Durch das vorbereitende Öffnen und/oder Schließen der Pneumatikventile ist es nicht mehr notwendig, diese unmittelbar bei Erreichen einer bestimmten Position des Kolbens bzw. der Kolbenstange zu schalten, wodurch sich ein größeres Zeitfenster für die Aktuierung des jeweiligen Pneumatikventils ergibt. Es kann beispielsweise ein pneumatisches Flexionsventil bereits geschlossen werden, wenn noch eine Extensionsbewegung durchgeführt wird und erwartet werden kann, dass zum Ende der Extensionsbewegung eine Flexionsunterstützung erfolgen soll. Zwischen dem Verbindungskanal und der Umgebungsatmosphäre kann eine strömungstechnische Verbindung ausgebildet sein, die über ein Füllventil verschlossen ist bzw. verschließbar ist. Über das Füllventil kann beispielsweise der Fülldruck in dem Pneumatiksystem angepasst werden.

Die orthopädietechnische Vorrichtung mit einem Oberteil und einem Unterteil, die über ein Gelenk schwenkbar aneinander gelagert sind, und einer Dämpfereinheit, wie sie oben beschrieben worden ist, sieht vor, dass diese Dämpfereinheit zwischen dem Oberteil und dem Unterteil angeordnet ist und einen Widerstand gegen eine Verschwenkung des Oberteils relativ zu dem Unterteil bereitstellt. Zusätzlich zu der Bereitstellung des Widerstandes aufgrund des hydraulischen Strömungswiderstandes und gegebenenfalls des pneumatischen Strömungswiderstandes ist es möglich, eine Unterstützung einer Bewegung aufgrund der vorhandenen Kompression einer Pneumatikkammer bereitzustellen.

Eine Weiterbildung sieht vor, dass dem zumindest einem Pneumatikventil ein Aktuator zugeordnet ist, der mit einer Steuerungseinrichtung gekoppelt ist, die mit Sensoren und/oder einer Bedieneinrichtung gekoppelt ist und auf Grundlage der Sensorwerte und/oder Befehle über die Bedieneinrichtung das zumindest eine Pneumatikventil verstellt. Die Betätigung des Pneumatikventils kann auch über eine rein mechanische Steuereinrichtung erfolgen, ohne dass ein Aktuator mit einem Energiespeicher vorhanden sein muss. Das Pneumatikventil kann über einen Mechanismus, z.B. bei einer Axiallast am Unterschenkel, schließen und, z.B. bei Wegfall der Axiallast, öffnen.

Eine Weiterbildung sieht vor, dass die orthopädietechnische Vorrichtung als Orthese oder Prothese einer unteren Extremität, insbesondere als ein künstliches Kniegelenk ausgebildet ist. Über die Dämpfereinheit ist es möglich, bei einer Ausgestaltung als künstliches Kniegelenk eine tiefere und somit physiologisch natürlichere Standphasenbeugung zu erreichen, da eine Standphasenstreckung durch die Pneumatikfeder unterstützt werden kann. Über die Kompression und Dekompression in der jeweiligen Pneumatikkammer kann Energie in der Dämpfereinheit gespeichert und wieder abgegeben werden, sodass die Dämpfereinheit gleichzeitig als Aktuator oder eine Bewegung unterstützende Einrichtung der orthopädietechnischen Vorrichtung dient. Die Federsteifigkeit der Pneumatikfeder ist einfach über den Druck innerhalb des Pneumatiksystems anzupassen. Die Anpassung kann beispielsweise an das Gewicht des Anwenders oder an die persönlichen Vorlieben des Anwenders erfolgen. Es ist kein zusätzlicher Steuerungsaufwand für die Pneumatikkomponente während der gefederten Standphase notwendig, da kein Umschalten von Ventilen beim Wechseln der Bewegungsrichtung erfolgt.

Nachfolgend werden Ausführungsbeispiele anhand der Figuren näher erläutert. Es zeigen:
- Figur 1 -: eine perspektivische Darstellung einer orthopädietechnischen Vorrichtung mit einer Dämpfereinheit;
- Figur 2 -: eine Variante der Figur 1 in Gestalt einer Orthese;
- Figur 3 -: ein Schaltbild einer ersten Variante der Dämpfereinheit;
- Figur 4 -: eine Variante mit einem beweglichen Hydraulikzylinder in einem Pneumatikzylinder; sowie
- Figur 5 -: eine Variante der Figur 3 mit zwei pneumatischen Stellventilen.

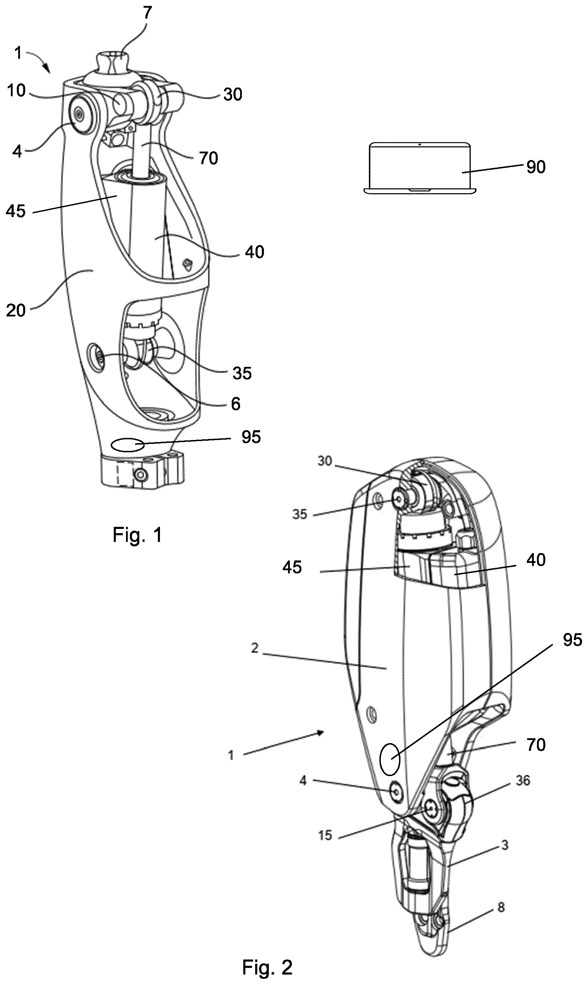

**In** der Figur 1 ist in einer perspektivischen Darstellung eine orthopädietechnische Gelenkeinrichtung 1 in Gestalt eines Prothesenkniegelenkes dargestellt. Die orthopädietechnische Gelenkeinrichtung 1 weist ein Oberteil 10 und ein Unterteil 20 auf, die schwenkbar um eine Gelenkachse 4 aneinander gelagert sind. Das Unterteil 20 ist als ein dreidimensionaler Hohlkörper ausgebildet, der einen Aktuator oder eine Dämpfereinheit 40 mit einer Kolbenstange 70 aufweist. Das Oberteil 10 weist an seinem proximalen Ende eine Einrichtung 7 zur Befestigung einer proximalen Komponente, beispielsweise eines Oberschenkelrohres oder eines Oberschenkelschaftes auf. Die Befestigungseinrichtung 7 ist in dem dargestellten Ausführungsbeispiel als Pyramidenadapter ausgebildet, andere Ausgestaltungen sind ebenfalls möglich. An dem Oberteil 10 ist weiterhin ein Kopf als Lagerstelle oder Lagersitz 30, der an einem proximalen Ende der Kolbenstange 70 angeordnet oder befestigt ist, schwenkbar um eine Achse oder einen Bolzen gelagert. Weiterhin ist das distale Ende der Dämpfereinheit 40 an einer distalen Lagerstelle oder einem distalen Lagersitz 35 schwenkbar um eine Achse 6 gelagert. Die Festlegung der Dämpfereinheit 40 sowohl an der distalen Lagerungsstelle 35 als auch an dem Kopf oder dem proximalen Lagersitz 30 kann lösbar, insbesondere über eine Schraubverbindung oder Schnappverbindung erfolgen.

Neben einer Ausgestaltung der orthopädietechnischen Gelenkeinrichtung 1 als ein Prothesenkniegelenk kann dieses auch als ein Orthesenkniegelenk oder eine andere Gelenkeinrichtung ausgebildet sein, wie sie in der Figur 2 gezeigt ist. Die Figur 2 zeigt in einer perspektivischen Darstellung eine alternative Ausgestaltung der orthopädietechnischen Einrichtung 1, nämlich als eine Orthesenkomponente. Ein Oberteil 10 in Gestalt eines Gehäuses zur Aufnahme der Dämpfereinheit 40 ist schwenkbar um eine Gelenkachse 4 an einem Unterteil 20 angeordnet, das über eine gelenkig daran befestigte Platte 8 beispielsweise an einer Orthesenschiene befestigt werden kann. Das Oberteil 10 wird ebenfalls an einer Orthesenschiene festgelegt. Die beiden nicht dargestellten Orthesenschienen werden dann an einer Gliedmaße befestigt, beispielsweise über Schalen, Gurte, Schnallen oder andere Einrichtungen zum Festlegen der Orthese an der Gliedmaße. Die orthopädietechnische Einrichtung 1 bildet somit ein Gelenk zwischen den beiden Orthesenschienen aus.

Die Dämpfereinheit 40, wie sie in den Figuren 1 und 2 dargestellt ist, weist eine hydraulische Dämpferkomponente und eine pneumatische Dämpferkomponente auf, deren Aufbau weiter unten erläutert wird.

Sowohl bei der Ausführungsform als Prothese als auch bei der Orthese ist die Dämpfereinheit 40 ist mit einer Steuerungseinrichtung 45 gekoppelt, in der die notwendigen Hardware- und Softwarekomponenten zur Verarbeitung von Sensordaten und zur Aktivierung und Deaktivierung von Aktuatoren angeordnet sind. Ebenfalls kann in der Steuerungseinrichtung 45 eine Energieversorgung bzw. ein Energiespeicher vorhanden sein. Ebenfalls sind der Steuerungseinrichtung 45 Schnittstellen zur Datenübertragung und/oder Energieübertragung zugeordnet. An den beiden Ausführungsformen gemäß den Figuren 1 und 2 sind Sensoren 95 schematisch dargestellt, die Daten aufnehmen, beispielsweise Raumlagedaten, Belastungsdaten, Winkeldaten, Temperaturen oder andere Parameter oder deren Veränderungen, auf deren Grundlage dann in der Steuerungseinrichtung 45 entsprechende Signale zur Aktivierung oder Deaktivierung von Aktuatoren zum Verstellen von Stellventilen aktiviert oder deaktiviert werden. Alternativ oder ergänzend können diese Ventile über eine Bedieneinrichtung 90, die exemplarisch als Computer dargestellt ist, verstellt oder eingestellt werden. Die Kommunikation mit der Steuerungseinrichtung 45 erfolgt drahtlos oder über eine Kabelverbindung. An der Dämpfereinheit 40 können ebenfalls Anschlüsse für einen Kompressor oder eine Pumpe vorhanden sein, um das Druckniveau in der Pneumatikkomponente an die Notwendigkeiten anpassen zu können. Alternativ kann eine Pumpe zum Befüllen und Aufladen der Dämpfereinheit 40 in der Steuerungseinrichtung 45 integriert sein.

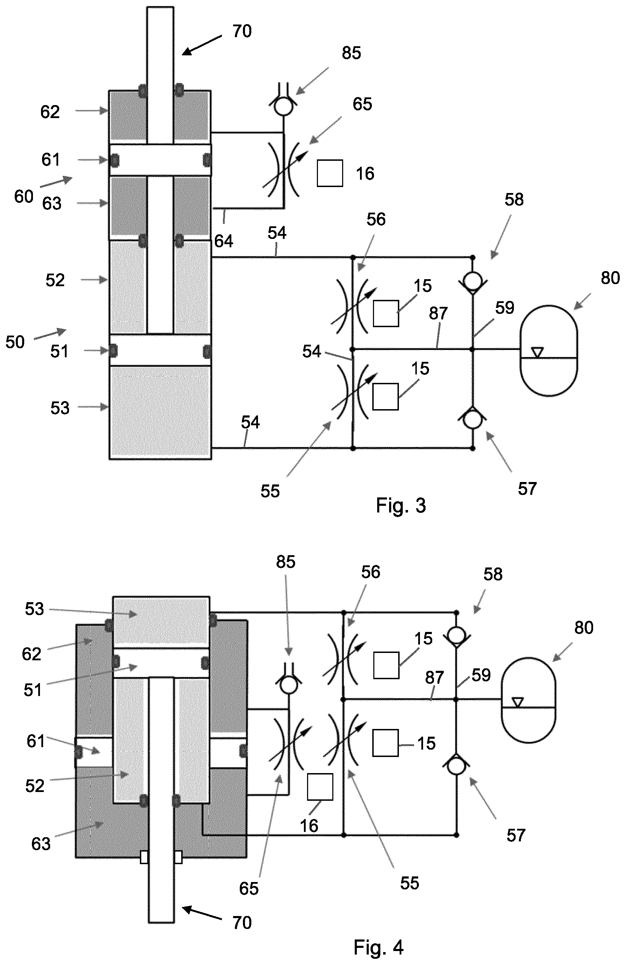

**In** der Figur 3 ist eine erste Variante der Dämpfereinheit 40 in einem nicht eingebauten Zustand in einer schematischen Darstellung gezeigt. Ein Hydraulikzylinder 50 weist einen verschieblich darin gelagerten Hydraulikkolben 51 auf, der an seinem Umfang eine Dichtung aufweist, sodass der Hydraulikzylinder 50 in zwei Hydraulikkammern 52 und 53 unterteilt ist. Über einen Hydraulikkanal 54, in dem zwei Stellventile 55, 56 angeordnet sind, findet ein Fluidaustausch zwischen den Hydraulikkammern 52, 53 statt. Den Stellventilen 55, 56 sind Aktuatoren 15 zugeordnet, um den Strömungsquerschnitt und damit den hydraulischen Widerstand zu verändern. Die Aktuatoren 15 sind mit der nicht dargestellten Steuerungseinrichtung 45 gekoppelt. Parallel zu dem Hydraulikkanal 54 mit den beiden Stellventilen 55, 56 ist ein Parallelkanal 59 ausgebildet, in dem zwei Rückschlagventile 57, 58 zueinander entgegengesetzt orientiert angeordnet sind. Zwischen den beiden Stellventilen 55, 56 sowie zwischen den beiden Rückschlagventilen 57, 58 ist ein hydraulischer Verbindungskanal 87 ausgebildet, der in ein Ausgleichsvolumen 80 mündet.

Oberhalb des Hydraulikzylinders 50 ist ein Pneumatikzylinder 60 angeordnet, durch den die Kolbenstange 70 hindurch geht. Ein Pneumatikkolben 61 ist an der Kolbenstange 70 angeordnet, der entsprechend dem Hydraulikkolben 51 eine umlaufende Dichtung aufweist und den Pneumatikzylinder 60 in zwei Pneumatikkammern 62, 63 teilt. Zwischen den beiden Pneumatikkammern 62, 63 ist ein Pneumatikkanal 64 ausgebildet, in dem ein Pneumatikventil 65 als Stellventil angeordnet ist, das über einen Aktuator 16, der mit der Steuerungseinrichtung 45 gekoppelt ist, verstellt werden kann. Ein Füllventil 85 ist mit dem Pneumatikkanal 64 verbunden und ermöglicht es, den Fülldruck innerhalb der Pneumatikkammern 62, 63 einzustellen. Über das Füllventil 85 kann auch unter Druck stehendes Gas abgelassen werden.

Die Kolbenstange 70 kann einteilig ausgebildet sein und durch den Pneumatikzylinder 60 hindurchgehen. Alternativ ist die Kolbenstange 70 zweiteilig ausgebildet und erstreckt sich beiderseits des Pneumatikkolbens 61 durch den Pneumatikzylinder 60. Der Übergang des verbindenden Abschnittes der Kolbenstange 70 zwischen dem Pneumatikkolben 61 und dem Hydraulikkolben 51 ist abgedichtet, sodass kein Hydraulikfluid in den Pneumatikzylinder 60 gelangt und umgekehrt kein Gas in den Hydraulikzylinder 50. Die Kolbenstange 70 geht nicht durch den Hydraulikzylinder 50 hindurch, der Hydraulikkolben 51 bildet den Abschluss der Kolbenstange 70, sodass die Kolbenstange 70 nur an einer Seite aus dem Hydraulikzylinder 50 hinausragt. Dadurch wird Bauhöhe der Dämpfereinheit 40 gespart, da die übereinander angeordneten Hydraulik- und Pneumatikzylinder 50, 60 nicht um eine durchragende Kolbenstange 70 ohne Anbindung an eine Komponente der orthopädietechnischen Vorrichtung verlängert werden muss.

Aufgrund der unterschiedlichen Volumina der Hydraulikkammern 52, 53 aufgrund der nur in einer Hydraulikkammer 52 befindlichen Kolbenstange 70 ist das Ausgleichsvolumen 80 vorgesehen. Darüber hinaus dient das Ausgleichsvolumen 80 als Vorratsvolumen für beispielsweise verdunstende Hydraulikflüssigkeit und kann zudem als Energiespeicher eingesetzt werden. Dazu ist in dem Ausgleichsvolume 80 eine Gasblase oder eine Feder, die auf einen Kolben drückt, angeordnet, so dass die Feder oder Gasblase bei jeden Einfahren der Kolbenstange komprimiert und bei jedem Ausfahren entspannt wird, wobei die Entspannung das Ausfahren der Kolbenstange unterstützt.

In der Figur 4 ist eine Variante des Aufbaus der Dämpfereinheit 40 gezeigt. Gleiche Bezugszeichen bezeichnen gleiche Komponenten. Der grundsätzliche Aufbau der hydraulischen Schaltung entspricht dem der Figur 3. Im Unterschied zu der übereinander oder hintereinander angeordneten Ausgestaltung des Hydraulikzylinders 50 und des Pneumatikzylinders 60 ist in der Figur 4 der Hydraulikzylinder 50 beweglich innerhalb des Pneumatikzylinders 60 angeordnet. An der Außenseite des Hydraulikzylinders 50 ist der Pneumatikkolben 61 angeordnet, der innerhalb des Pneumatikzylinders 60 zusammen mit dem Hydraulikzylinder 50 bewegt wird. Der Hydraulikzylinder 50 ragt nach oben aus dem Pneumatikzylinder 60 hinaus und ist an der Durchtrittsstelle abgedichtet. Ebenso ragt die Kolbenstange 70 aus dem Hydraulikzylinder 50 heraus, die Abdichtung ist ebenfalls eingezeichnet. Der Pneumatikzylinder 60 ist fest mit der Kolbenstange 70 verbunden. Das Oberteil der orthopädietechnischen Vorrichtung ist entweder an der Kolbenstange 70 oder dem Hydraulikzylinder 50 angeordnet, das Unterteil an der jeweils anderen Komponente. Auch hier ist ein Ausgleichsvolumen 80 vorgesehen, anders als bei der Ausgestaltung gemäß der Figur 3 ergibt sich bei dieser Ausführungsform ein unterschiedliches Volumen der Pneumatikkammern 62, 63.

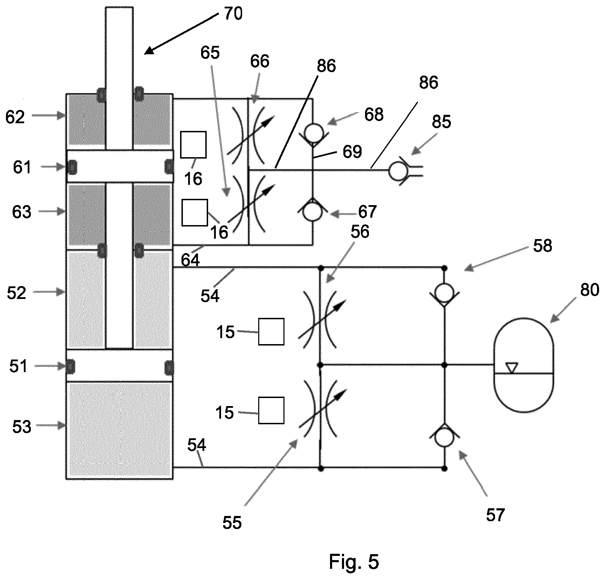

In der Figur 5 ist eine weitere Variante dargestellt, die im Wesentlichen dem Aufbau der Figur 3 entspricht. Die pneumatische Verschaltung entspricht der hydraulischen Verschaltung, das heißt, dass zwei Pneumatikventile 65, 66 vorhanden sind, die über Aktuatoren 16 verstellt werden können. Zwei Rückschlagventile 67, 68 sind in einem Parallelkanal 69 entgegengesetzt orientiert angeordnet, ein Verbindungskanal 86 stellt eine Verbindung mit dem Füllventil 85 her. Der Verbindungskanal 86 ist zwischen den Stellventilen oder Pneumatikventilen 65, 66 und den Rückschlagventilen 67, 68 angeordnet.

Durch die Ausgestaltung der Dämpfereinheit 40 mit einer Pneumatikkomponente und einer Hydraulikkomponente und der Ausgestaltung der Hydraulikkomponente mit einer nicht durchgehenden Kolbenstange kann das notwendige Ausgleichsvolumen 80 dazu verwendet werden, während des Betriebes auftretende Volumenänderungen aufgrund von Wärmeausdehnungen oder Ölverdunstungen zu kompensieren. Dadurch wird neben der Einsparung des Bauraumes durch den Verzicht einer durchgehenden Kolbenstange 70 die Betriebssicherheit erhöht. Darüber hinaus kann das Ausgleichsvolumen 80 mit Druck beaufschlagt werden, entweder mit pneumatischem Druck oder mechanischem Federdruck, sodass eine Vorbringerwirkung über das Ausgleichsvolumen 80 durch die Hydraulikkomponente der Dämpfereinheit 40 erreicht werden kann.

Die Pneumatikkomponente der Dämpfereinheit 40 weist aufgrund der durchgehenden Kolbenstange 70 oder der zumindest auf beiden Seiten des Pneumatikkolbens 61 ausgebildeten Kolbenstange 70 gleichgroße, wirksame Kolbenflächen auf, sodass sich bei einer Verlagerung des Pneumatikkolbens 61 gemäß der Ausführungsform der Figuren 3 und 5 dadurch eine gleichgroße Volumenänderung in den beiden Pneumatikkammern 62, 63 ergibt. Die Volumenänderung ist betragsmäßig gleich, die Volumenverringerung der einen Kammer führt zu einer entsprechenden Volumenvergrößerung der anderen Kammer. Dadurch ist es möglich, die Pneumatik allein durch Öffnung eines einzigen Pneumatikventils 65 wirkungslos zu schalten. Bei einem geöffneten Pneumatikventil 65 erfordert die Verschiebung des Luftvolumens oder Gasvolumens innerhalb der Pneumatikkammern 62, 63 keine oder nur eine zu vernachlässigende Kraft, sodass keine Dämpfungswirkung stattfindet. Das Pneumatikventil 65 kann in jeder beliebigen Stellung des Pneumatikzylinders 61 geschlossen werden, sodass sich dadurch eine Pneumatikfeder durch den Pneumatikzylinder 60 ausbilden lässt. Der Nullpunkt dieser Feder ist beliebig wählbar. Aufgrund der gleichgroßen Verdrängungsvolumina in den Pneumatikkammern 62, 63 kann das Pneumatiksystem zudem mit sehr hohen Drücken befüllt werden, sodass zum Beispiel bei dem Einsatz in künstlichen Kniegelenken ein steuerbarer Einfedereffekt und eine Rückstellunterstützung erreicht werden kann.

Weiterhin kann durch das Verstellen des Ventils oder der Ventile der Einsatz einer Pneumatikfeder in unterschiedlichen Bewegungssituationen oder Verstellsituationen erreicht werden. Bei einem künstlichen Kniegelenk kann die Federwirkung durch das Schließen des Pneumatikventils an der vorgesehenen Stellung des Pneumatikkolbens 61 bei einer relativ geringen Flexion von 4° bis 8° Flexionswinkel bewirkt werden, um eine Standphasenextension zu unterstützen. Ebenso ist es möglich, eine Schwungphasenumkehr bei einem Flexionswinkel von 30° oder mehr in der Schwungphase zu bewirken, indem das Pneumatikventil entsprechend eingestellt wird.

Sofern eine Ausschubwirkung des Pneumatikkolbens bei einem offenen Pneumatikventil akzeptiert werden kann, ist die Ausgestaltung gemäß Figur 4 aufgrund der kompakten Bauweise und der kürzeren Bauhöhe der Dämpfereinheit vorteilhaft. Aufgrund der unterschiedlichen Kolbenstangendurchmesser ist eine vergleichsweise hohe Vorbringerwirkung zu erwarten.

Bei einer Verschaltung der Pneumatikkomponente wie in Figur 5 gezeigt, lässt sich ein antizipierendes Positionieren der Pneumatikventile 65, 66 erreichen. Die Stellventile 65, 66 müssen nicht instantan geschaltet werden, sondern können vorbereitend in die jeweils gewünschte Stellung gebracht werden. Beispielsweise kann während der terminalen Standphase bei einem Einsatz in einem künstlichen Kniegelenk das Flexionsstellventil 65 geöffnet werden. **In** der terminalen Standphase wird der Pneumatikkolben dabei in die obere Arbeitspneumatikkammer 62 gedrückt, während das pneumatische Extensionsstellventil 66 geschlossen bleibt. Die pneumatische Extensionsfeder unterstützt die Schwungphaseneinleitung, das heißt, dass der Pneumatikkolben 61 nach unten gedrückt wird. Sobald die Pneumatikfeder entspannt ist, also gleicher Druck in den beiden Pneumatikkammern 62, 63 herrscht und der Pneumatikkolben 61 sich weiter absenkt, strömt das Gas über das Flexionsstellventil 65 und das Flexionsrückschlagventil 68 zurück in die obere Pneumatikkammer 62.

## Patentansprüche

1. Dämpfereinheit (40) mit einem Hydraulikzylinder (50) mit einem verschieblich darin gelagerten Hydraulikkolben (51), der mit einer Kolbenstange (70) gekoppelt ist und den Hydraulikzylinder (50) in zwei Hydraulikkammern (52, 53) unterteilt, die über zumindest einen Hydraulikkanal (54) miteinander strömungstechnisch verbunden sind, und mit einem Pneumatikzylinder (60) mit einem verschieblich darin gelagerten Pneumatikkolben (61), der mit der Kolbenstange (70) gekoppelt ist und den Pneumatikzylinder (60) in zwei Pneumatikkammern (62, 63) unterteilt, die über zumindest einen Pneumatikkanal (64) miteinander strömungstechnisch verbunden sind, wobei der Betrag der Volumenänderung der Hydraulikkammern (52, 53) bei einer Verlagerung des Hydraulikkolbens (51) verschieden ist und die Hydraulikkammern (52, 53) mit einem Ausgleichsvolumen (80) strömungstechnisch gekoppelt sind, **dadurch gekennzeichnet, dass** der Betrag der Volumenänderung der Pneumatikkammern (62, 63) bei einer Verlagerung des Pneumatikkolbens (61) gleich ist.

2. Dämpfereinheit (40) nach Anspruch 1, **dadurch gekennzeichnet, dass** der Pneumatikkolben (61) an der Kolbenstange (70) angeordnet ist und die Kolbenstange (70) den Pneumatikzylinder (60) durchragt.

3. Dämpfereinheit (40) nach Anspruch 1, **dadurch gekennzeichnet, dass** der Pneumatikkolben (61) an dem Hydraulikzylinder (50) befestigt und der Hydraulikzylinder (50) beweglich in dem Pneumatikzylinder (60) angeordnet ist.

4. Dämpfereinheit (40) nach einem der voranstehenden Ansprüche, **dadurch gekennzeichnet, dass** in dem Hydraulikanal (54) zumindest ein schaltbares oder verstellbares Hydraulikventil (55, 56) angeordnet ist.

5. Dämpfereinheit (40) nach einem der voranstehenden Ansprüche, **dadurch gekennzeichnet, dass** in dem Pneumatikkanal (64) zumindest ein schaltbares oder verstellbares Pneumatikventil (65, 66) angeordnet ist.

6. Dämpfereinheit (40) nach einem der voranstehenden Ansprüche, **dadurch gekennzeichnet, dass** in dem Pneumatikkanal (64) zwei Pneumatikventile (65, 66) angeordnet sind, zu denen zwei einander entgegengesetzt geschaltete Rückschlagventile (67, 68) in einem Parallelkanal (69) angeordnet sind, wobei der Parallelkanal (69) mit dem Pneumatikkanal (64) über einen Verbindungskanal (86) zwischen den Pneumatikventilen (65, 66) und den Rückschlagventilen (67, 68) verbunden ist.

7. Dämpfereinheit nach Anspruch 6, **dadurch gekennzeichnet, dass** der Verbindungskanal (86) eine strömungstechnische Verbindung zu der Umgebungsatmosphäre bereitstellt und über ein Füllventil (85) verschlossen ist.

8. Orthopädietechnische Vorrichtung mit einem Oberteil (10) und einem Unterteil (20) die über ein Gelenk (30) schwenkbar aneinander gelagert sind, und einer Dämpfereinheit (40) nach einem der voranstehenden Ansprüche, die zwischen dem Oberteil (10) und dem Unterteil (20) angeordnet ist und einen Widerstand gegen eine Verschwenkung des Oberteil (10) relativ zu dem Unterteil (20) bereitstellt.

9. Orthopädietechnische Vorrichtung nach Anspruch 8, **dadurch gekennzeichnet, dass** dem zumindest einem Pneumatikventil (65, 66) ein Aktuator (16) zugeordnet ist, der mit einer Steuerungseinrichtung (45) gekoppelt ist, die mit Sensoren (95) und/oder einer Bedieneinrichtung (90) gekoppelt ist und auf Grundlage der Sensorwerte und/oder Befehle über die Bedieneinrichtung (90) das zumindest eine Pneumatikventil (65, 66) verstellt.

## Claims

1. A damper unit (40) having a hydraulic cylinder (50) with a hydraulic piston (51) which is mounted displacably therein and which is coupled to a piston rod (70) and divides the hydraulic cylinder (50) into two hydraulic chambers (52, 53) which are fluidically interconnected via at least one hydraulic channel (54), and having a pneumatic cylinder (60) with a pneumatic piston (61) which is mounted displacably therein and which is coupled to the piston rod (70) and divides the pneumatic cylinder (60) into two pneumatic chambers (62, 63) which are fluidically interconnected via at least one pneumatic channel (64), wherein the value of the volume change of the hydraulic chambers (52, 53) during a movement of the hydraulic piston (51) differs and the hydraulic chambers (52, 53) are fluidically coupled to a compensating volume (80), **characterized in that** the value of the volume change of the pneumatic chambers (62, 63) during a movement of the pneumatic piston (61) is identical.

2. The damper unit (40) as claimed in claim 1, **characterized in that** the pneumatic piston (61) is arranged on the piston rod (70) and the piston rod (70) projects through the pneumatic cylinder (60).

3. The damper unit (40) as claimed in claim 1, **characterized in that** the pneumatic piston (61) is fastened to the hydraulic cylinder (50) and the hydraulic cylinder (50) is arranged movably in the pneumatic cylinder (60).

4. The damper unit (40) as claimed in one of the preceding claims, **characterized in that** at least one switchable or adjustable hydraulic valve (55, 56) is arranged in the hydraulic channel (54).

5. The damper unit (40) as claimed in one of the preceding claims, **characterized in that** at least one switchable or adjustable pneumatic valve (65, 66) is arranged in the pneumatic channel (64).

6. The damper unit (40) as claimed in one of the preceding claims, **characterized in that** in the pneumatic channel (64) there are arranged two pneumatic valves (65, 66) with respect to which two mutually oppositely switched check valves (67, 68) are arranged in a parallel channel (69), the parallel channel (69) being connected to the pneumatic channel (64) via a connecting channel (86) between the pneumatic valves (65, 66) and the check valves (67, 68).

7. The damper unit as claimed in claim 6, **characterized in that** the connecting channel (86) provides a fluidic connection to the surrounding atmosphere and is closed via a filling valve (85).

8. An orthopedic device having an upper part (10) and a lower part (20) which are mounted pivotably on each other via a joint (30), and having a damper unit (40) as claimed in one of the preceding claims which is arranged between the upper part (10) and the lower part (20) and provides resistance to pivoting of the upper part (10) relative to the lower part (20).

9. The orthopedic device as claimed in claim 8, **characterized in that** the at least one pneumatic valve (65, 66) is assigned an actuator (16) which is coupled to a control device (45), which is coupled to sensors (95) or to an operator control device (90) and adjusts the at least one pneumatic valve (65, 66) on the basis of the sensor values and/or commands via the operator control device (90).

## Revendications

1. Unité d'amortissement (40) comprenant un cylindre hydraulique (50) pourvu d'un piston hydraulique (51) monté de manière coulissante dans celui-ci, qui est couplé à une tige de piston (70) et qui subdivise le cylindre hydraulique (50) en deux chambres hydrauliques (52, 53) qui sont reliées fluidiquement l'une à l'autre par au moins un canal hydraulique (54), et comprenant un cylindre pneumatique (60) pourvu d'un piston pneumatique (61) monté de manière coulissante dans celui-ci, qui est couplé à la tige de piston (70) et qui subdivise le cylindre pneumatique (60) en deux chambres pneumatiques (62, 63) qui sont reliées fluidiquement l'une à l'autre par au moins un canal pneumatique (64), la valeur de la variation de volume des chambres hydrauliques (52, 53) lors d'un déplacement du piston hydraulique (51) étant différente, et les chambres hydrauliques (52, 53) étant couplées fluidiquement à un volume de compensation (80),
**caractérisée en ce que** la valeur de la variation de volume des chambres pneumatiques (62, 63) lors d'un déplacement du piston pneumatique (61) est identique.

2. Unité d'amortissement (40) selon la revendication 1,
**caractérisée en ce que** le piston pneumatique (61) est disposé sur la tige de piston (70), et la tige de piston (70) traverse le cylindre pneumatique (60).

3. Unité d'amortissement (40) selon la revendication 1,
**caractérisée en ce que** le piston pneumatique (61) est fixé au cylindre hydraulique (50), et le cylindre hydraulique (50) est disposé de manière mobile dans le cylindre pneumatique (60).

4. Unité d'amortissement (40) selon l'une des revendications précédentes, **caractérisée en ce qu'**au moins une valve hydraulique (55, 56) commutable ou réglable est disposée dans le canal hydraulique (54).

5. Unité d'amortissement (40) selon l'une des revendications précédentes, **caractérisée en ce qu'**au moins une valve pneumatique (65, 66) commutable ou réglable est disposée dans le canal pneumatique (64).

6. Unité d'amortissement (40) selon l'une des revendications précédentes, **caractérisée en ce que** deux valves pneumatiques (65, 66) sont disposées dans le canal pneumatique (64), par rapport auxquelles deux clapets anti-retour (67, 68) montés en sens inverse l'un de l'autre sont disposés dans un canal parallèle (69), le canal parallèle (69) étant relié au canal pneumatique (64) par l'intermédiaire d'un canal de liaison (86) entre les valves pneumatiques (65, 66) et les clapets anti-retour (67, 68).

7. Unité d'amortissement selon la revendication 6,
**caractérisée en ce que** le canal de liaison (86) assure une liaison fluidique avec l'atmosphère ambiante et est fermé par une valve de remplissage (85).

8. Dispositif orthopédique comprenant une partie supérieure (10) et une partie inférieure (20) qui sont montées pivotantes l'une par rapport à l'autre par l'intermédiaire d'une articulation (30), et comprenant une unité d'amortissement (40) selon l'une des revendications précédentes, qui est disposée entre la partie supérieure (10) et la partie inférieure (20) et qui assure une résistance au pivotement de la partie supérieure (10) par rapport à la partie inférieure (20).

9. Dispositif orthopédique selon la revendication 8,
**caractérisé en ce qu'**un actionneur (16) est associé à ladite au moins une valve pneumatique (65, 66), lequel est couplé à un dispositif de commande (45) qui est couplé à des capteurs (95) et/ou à un dispositif de manipulation (90) et qui déplace ladite au moins une valve pneumatique (65, 66) en se basant sur les valeurs des capteurs et/ou sur des instructions fournies par le dispositif de manipulation (90).
